# EUROPEAN PATENT APPLICATION

(11) **EP 4 309 641 A1**
(43) Date of publication of application: **24.01.2024**
(21) Application number: 22185663.6
(22) Date of filing: 19.07.2022
(51) Int. Cl.: A61K 9/00, A61K 9/10, A61K 47/26, A61K 47/32

(54) **REPARIXIN LIQUID SUSPENSIONS**

(71) Applicant: Dompe' Farmaceutici S.P.A., 20122 Milan (IT)
(72) Inventor: ROMEO, Tiziana, 60100 L'Aquila (IT); ALBERTINI, Barbara, 60100 l'Aquila (IT); DRAGANI, Maria Concetta, 67100 L'Aquila (IT)
(74) Representative: Mauri, Elisabetta Maria Ester

(57) **Abstract**

The present invention relates to stable, ready-to-use liquid suspensions of reparixin and to uses thereof.

## Description

### FIELD OF THE INVENTION

The present invention relates to liquid suspensions comprising reparixin.

### STATE OF THE ART

Reparixin, also known as R-(-)-2-(4-isobutylphenyl)propionyl methansulfonamide and repertaxin, is a non competitive allosteric inhibitor of the interleukin 8 (IL-8) receptors, CXCR1 and CXCR2 (Bertini R, et al., Proc Natl Acad Sci. 2004;101(32):11791-6). Reparixin has a therapeutic potential in preventing or treating pathologies where the activation of this pathway is detrimental.

For instance, IL-8 is involved in neutrophil infiltration in pathologies such as psoriasis (B. J. Nickoloff et al., Am. J. Pathol., 1991, 138, 129), rheumatoid arthritis (M. Selz et al., J. Clin. Invest. 1991, 87, 463), ulcerative colitis (Y. R. Mahkla et al., Clin. Sci., 1992, 82, 273), acute respiratory distress syndrome (ARDS), idiopathic fibrosis (P. C. Carré et al., J. Clin. Invest,. 1991, 88, 1802, and E.J. Miller et al., Am. Rev. Respir. Dis. 1992, 146:427-432) and glomerulonephritis (T. Wada et al., J. Exp. Med., 1994, 180,1135).

*In vitro* and preclinical small animal studies have demonstrated that binding of reparixin to CXCR1/CXCR2 can prevent leukocyte recruitment and activation of inflammation. Action of reparixin includes inhibition of neutrophil stimulation and downstream effects of neutrophils, such as NETosis, the formation of NETs, during an inflammatory response (Cheng OZ, Palaniyar N., Front Immunol.2013;4:1).

The dose, safety, and pharmacokinetics of reparixin have been investigated in clinical studies of patients with metastatic breast cancer (Schott AF, et al., Clin Cancer Res. 2017;23(18):5358-65; Goldstein LJ et al., Breast Cancer Res Treat. 2021;190(2):265-75).

A phase 2, multicenter, open-label, randomized, clinical trial to assess the efficacy and safety of reparixin compared with the available treatments considered as standard of care (SOC) in hospitalized patients with severe COVID-19 pneumonia was carried out from May 5, 2020 until November 27, 2020, showing that treatment with reparixin led to improved clinical outcomes in patients with severe COVID-19 pneumonia compared with the standard of care (Landoni G. et al., Infect Dis Ther 2022, 26: 1-16).

At the moment, reparixin has been formulated only in form of tablets for oral administration or solution for i.v. administration. However, these types of administrations are invasive and/or are not suitable for achieving satisfactory patient compliance, especially in case of pediatric patients or patients who are unable to swallow solid tablets. Consequently, there is a need to develop new pharmaceutical dosage forms containing reparixin that are able to overcome these problems and can be administered to subjects of all conditions and ages. To this aim, suspensions are ideal candidates.

Suspensions are pharmaceutical dosage forms with two phases: an external liquid phase, also known as continuous phase or dispersion medium, and an internal phase, also known as dispersed phase, that contains particulate matter insoluble in the external phase. Suspensions are recognized as heterogeneous dispersed systems and most pharmaceutical suspensions have an aqueous dispersion medium.

The formulation and dispensation of this type of dosage forms is associated with several advantages: efficient dispense of hydrophobic drugs; avoidance of the use of cosolvents; masking of unpleasant taste; offering resistance to degradation of drugs due to hydrolysis, oxidation or microbial activity; easy ingesting for young or elderly patients. In addition, higher concentration of drugs can be incorporated in suspensions than in solutions (Doye, P. et al., Int J Curr Pharm Sci 2017, 9, 8).

The main challenge in the development of suspensions is to guarantee their physical stability over time for assuring content uniformity. Indeed, suspensions are thermodynamically unstable systems due to the free energy of their internal phase, which produces particle aggregation and sedimentation in order to decrease the energy of the system. Several aspects must be considered when formulating a suspension.

First of all, based on the particle size of the internal phase, suspensions can be classified in two groups: suspensions characterized by an internal phase with particle size greater than ~0.5 µm, known as coarse suspensions, and suspensions characterized by an internal phase with particle size smaller than 0.5 µm, known as colloidal dispersions (Edman, P., Journal of Aerosol Medicine 1994, 7, S-3-S-6). Based on the type of suspension, different parameters must be considered to ensure stability.

The preparation of suspensions containing reparixin is particularly difficult due to the specific features of this active pharmaceutical ingredient (API).

First of all, reparixin is produced in form of powder composed of particles having large particle size. Therefore, potential suspensions containing reparixin are coarse suspensions.

In case of coarse suspensions, the internal phase with the API tends to settle under gravitational force and cause sedimentation phenomena due to its particle size. The increase of zeta potential in terms of absolute value produces an increase of the sedimentation rate. This suggests that sedimentation is driven by gravitational forces rather than by electrostatic interactions, which are insufficient to provide stability and kinetic stabilization because of the particle size of the internal phase. For this reason, the zeta potential value of coarse suspensions is usually set around zero for ensuring physical stability. This value of zeta potential corresponds to a condition where particles are surrounded by a hydration layer which hides their charge and, in case the particles aggregate and sediment, is able to prevent the formation of a compact sediment and instead promotes the formation of a loose sediment that can be resuspended easily by shaking manually. For adjusting the zeta potential value of suspensions, ionic or nonionic surfactants and different type of electrolytes (e.g. salts) are commonly employed. For instance, PEG (polyethylene glycol) is included in many commercial suspensions for this purpose.

Furthermore, reparixin has low wettability, which complicates its formulation into a stable liquid suspension. In fact, the wettability of the dispersed particles is important for ensuring homogeneity and content uniformity (Kwok, D.Y. et al., Advances in Colloid and Interface Science 1999, 81, 167-249). Wettability is the tendency of a fluid to spread on or adhere to a solid surface when they are immiscible; the consequence of a poor wettability of a powder solid is its floating on the surface of liquid medium. A poor wettability of the internal phase is mainly due to the high interfacial tension between the dispersed particles and the dispersion medium and the reduction of interfacial tension increases the stability of the suspension. When formulating a suspension, a common way to improve the wettability of solids is the use of wetting agents such as surfactants, able to reduce the solid-liquid interfacial tension.

Another requirement necessary for ensuring stability of suspensions is the presence of so-called yield stress (τ0), which is a material property and can be defined as the stress corresponding to the yield point at which the material begins to flow or deform plastically. In light of the above, there is clearly a need to develop suspensions containing reparixin with satisfactory features. However, this is particularly challenging for this API, in view of the reasons discussed above.

### SUMMARY OF THE INVENTION

The present inventors have developed stable liquid suspensions of reparixin.

In particular, the inventors have found that the use in the formulation of sucrose as sweetener and polyvinylpyrrolidone as wetting agent allows to produce stable, ready-to-use liquid suspensions of reparixin.

Specifically, the inventors have suprisingly found that the type and amount of sweetener included in the formulation to mask the unpleasant taste of reparixin influences the zeta potential value and, consequently, the stability of the suspension. Furthermore, the inventors have identified that the use of polyvinylpyrrolidone as wetting agent allows to obtain a satisfactory wettability of reparixin.

The inventors have also identified the type and range of viscosity agent to be used in combination with the above-mentioned excipients to achieve a stable, ready-to-use liquid suspension of reparixin.

Accordingly, an object of the present invention is a liquid suspension comprising:
- reparixin in an amount between 2% w/w and 13% w/w,
- xanthan gum in an amount between 0.15% w/w and 0.3% w/w,
- sucrose in an amount between 25% w/w and 55% w/w,
- polyvinylpyrrolidone in an amount between 1.8% w/w and 3.2% w/w,
- a water insoluble polymer in an amount between 2.25% w/w and 2.75% w/w.

A further object of the present invention is a liquid suspension according to the first object of the invention for use in the treatment of a disease or condition selected from psoriasis, rheumatoid arthritis, ulcerative colitis, acute respiratory distress syndrome (ARDS), idiopathic fibrosis, glomerulonephritis, COVID-19.

A further object of the present invention is a liquid suspension according to the first object of the invention for use in the prevention of diabetes or in delaying the onset and the progression of diabetes.

A further object of the present invention is a liquid suspension according to the first object of the invention for use in the prevention and/or treatment of seizures.

### BRIEF DESCRIPTION OF THE FIGURES

Figure 1 shows the frequency sweep profile of suspension 82.
Figure 2 shows the frequency sweep profile of suspension 98.
Figure 3 shows the frequency sweep profile of suspension 105.
Figure 4 shows the frequency sweep profile of suspension 106.
Figure 5 shows the frequency sweep profile of suspension 107.
Figure 6 shows the frequency sweep profile of suspension 165.
Figure 7 shows the frequency sweep profile of suspension 159.
Figure 8 shows the frequency sweep profile of suspension 160.
Figure 9 shows the frequency sweep profile of suspension 166.
Figure 10 shows the frequency sweep profile of suspension 168.

### DEFINITIONS

The term "antifoaming agent" as used herein refers to a chemical additive that reduces and/or hinders the formation of foam in liquid formulations, e.g., liquid suspensions, by decreasing the liquid-air surface tension.

The term "anticaking agent" as used herein refers to excipients able to help the suspension's stabilization and improve the appearance of the final formulation. These agents are generally chemical compounds insoluble in water and they include inorganic solids, insoluble cellulose derivatives and insoluble synthetic polymers.

The term "wetting agent" as used herein refers to excipients able to reduce the API-water interfacial tension improving the wettability and spreadability of the suspended API.

As used herein with reference to a component of the liquid suspension according to the invention, "% w/w" indicates the grams of that component contained in 100 grams of the suspension. For instance, the wording "reparixin in an amount of 5% w/w" indicates that 5 grams of reparixin are contained in 100 grams of the suspension.

As used herein, the term "Avicel CL-611" indicates a spray-dried blend of microcrystalline cellulose and sodium carboxymethylcellulose, wherein sodium carboxymethylcellulose is present in an amount between 11.3% w/w and 18.8% w/w.

It is understood that terms as "suspending" and "dispersing", or "suspended" and "dispersed" can be used interchangeably in the context of the present description.

### DETAILED DESCRIPTION OF THE INVENTION

As will be explained more in details in the experimental section, the inventors have identified the quali-quantitative composition that allows to produce stable liquid suspensions of reparixin.

In particular, it was unexpectedly found that the type and amount of sweetener included in a liquid suspension of reparixin to mask the unpleasant taste of this API affect the zeta potential value and the rheological properties of the produced suspension and, therefore, its stability. Specifically, based on the results reported in the experimental section, sucrose in an amount between 25% w/w and 55% w/w was selected as sweetener to be included in liquid suspensions of reparixin to achieve the desired stability.

Also, the inventors have selected polyvinylpyrrolidone as wetting agent to be included in liquid suspensions of reparixin in order to achieve a satisfactory wettability of this API.

Furthermore, the inventors have selected the type/amounts of the viscosity agent (xanthan gum) and anticaking agent (water insoluble polymers) that are to be used in combination with sucrose and polyvinylpyrrolidone to obtain stable liquid suspensions of reparixin.

Accordingly, a first object of the invention is a liquid suspension comprising:
- reparixin in an amount between 2% w/w and 13% w/w, preferably between 3% w/w and 13% w/w, more preferably between 4% w/w and 13% w/w, more preferably between 4.5% w/w and 12.5% w/w, more preferably between 4.8% w/w and 12.2% w/w, even more preferably between 4.9% w/w and 12.1% w/w, most preferably between 5% w/w and 12% w/w,
- xanthan gum in an amount between 0.15% w/w and 0.3% w/w, preferably between 0.18% w/w and 0.27% w/w, more preferably between 0.18% w/w and 0.23% w/w, even more preferably between 0.19% w/w and 0.21% w/w, most preferably in an amount of 0.2% w/w,
- sucrose in an amount between 25% w/w and 55% w/w, preferably between 27% w/w and 53% w/w, even more preferably between 29% w/w and 51% w/w, most preferably between 30% w/w and 50% w/w,
- polyvinylpyrrolidone, preferably polyvinylpyrrolidone K 30, in an amount between 1.8% w/w and 3.2% w/w, preferably between 1.9% w/w and 3.1% w/w, more preferably between 2% w/w and 3% w/w,
- a water insoluble polymer in an amount between 2.25% w/w and 2.75% w/w.

Reparixin (CAS No.: 266359-83-5), which is also known as R-(-)-2-(4-isobutylphenyl)propionyl methansulfonamide and repertaxin, is the compound having the following chemical formula:

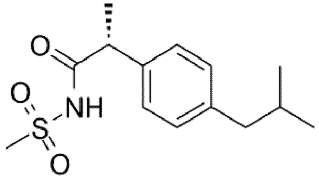

In an embodiment, the liquid suspension according to the first object of the invention further comprises a buffering agent, preferably in an amount between 0.1% w/w and 3% w/w, and/or an antifoaming agent, preferably in an amount between 0.005% w/w and 5% w/w, and/or a preservative agent, preferably in an amount between 0.01% w/w and 0.5% w/w.

In an embodiment, the liquid suspension according to the first object of the invention consists of:
- reparixin in an amount between 2% w/w and 13% w/w, preferably between 3% w/w and 13% w/w, more preferably between 4% w/w and 13% w/w, more preferably between 4.5% w/w and 12.5% w/w, more preferably between 4.8% w/w and 12.2% w/w, even more preferably between 4.9% w/w and 12.1% w/w, most preferably between 5% w/w and 12% w/w,
- xanthan gum in an amount between 0.15% w/w and 0.3% w/w, preferably between 0.18% w/w and 0.27% w/w, more preferably between 0.18% w/w and 0.23% w/w, even more preferably between 0.19% w/w and 0.21% w/w, most preferably in an amount of 0.2% w/w,
- sucrose in an amount between 25% w/w and 55% w/w, preferably between 27% w/w and 53% w/w, even more preferably between 29% w/w and 51% w/w, most preferably between 30% w/w and 50% w/w,
- polyvinylpyrrolidone, preferably polyvinylpyrrolidone K 30, in an amount between 1.8% w/w and 3.2% w/w, preferably between 1.9% w/w and 3.1% w/w, more preferably between 2% w/w and 3% w/w,
- a water insoluble polymer in an amount between 2.25% w/w and 2.75% w/w,
- a buffering agent, preferably in an amount between 0.1% w/w and 3% w/w,
- an antifoaming agent, preferably in an amount between 0.005% w/w and 5%,
- a preservative agent, preferably in an amount between 0.01% and 0.5%.
- an aqueous solvent, preferably water, in an amount up to 100% w/w.

Preferably, the amount of reparixin in said liquid suspension is 2% w/w, 2.1% w/w, 2.2% w/w, 2.3% w/w, 2.4% w/w, 2.5% w/w, 2.6% w/w, 2.7% w/w, 2.8% w/w, 2.9% w/w, 3% w/w, 3.1% w/w, 3.2% w/w, 3.3% w/w, 3.4% w/w, 3.5% w/w, 3.6% w/w, 3.7% w/w, 3.8% w/w, 3.9% w/w, 4% w/w, 4.1% w/w, 4.2% w/w, 4.3% w/w, 4.4% w/w, 4.5% w/w, 4.6% w/w, 4.7% w/w, 4.8% w/w, 4.9 % w/w, 5% w/w, 5.1% w/w, 5.2% w/w, 5.3% w/w, 5.4% w/w, 5.5% w/w, 5.6% w/w, 5.7% w/w, 5.8% w/w, 5.9% w/w, 6% w/w, 6.1% w/w, 6.2% w/w, 6.3% w/w, 6.4% w/w, 6.5% w/w, 6.6% w/w, 6.7% w/w, 6.8% w/w, 6.9% w/w, 7% w/w, 7.1% w/w, 7.2% w/w, 7.3% w/w, 7.4% w/w, 7.5% w/w, 7.6% w/w, 7.7% w/w, 7.8% w/w, 7.9% w/w, 8% w/w, 8.1% w/w, 8.2% w/w, 8.3% w/w, 8.4% w/w, 8.5% w/w, 8.6% w/w, 8.7% w/w, 8.8% w/w, 8.9% w/w, 9% w/w, 9.1% w/w, 9.2% w/w, 9.3% w/w, 9.4% w/w, 9.5% w/w, 9.6% w/w, 9.7% w/w, 9.8% w/w, 9.9% w/w, 10% w/w, 10.1 % w/w, 10.2% w/w, 10.3% w/w, 10.4% w/w, 10.5% w/w, 10.6% w/w, 10.7% w/w, 10.8% w/w, 10.9% w/w, 11 % w/w, 11.1 % w/w, 11.2% w/w, 11.3%w/w, 11.4% w/w, 11.5% w/w, 11.6% w/w, 11.7% w/w, 11.8% w/w, 11.9% w/w, 12% w/w, 12.1% w/w, 12.2% w/w, 12.3% w/w, 12.4% w/w, 12.5% w/w, 12.6% w/w, 12.7% w/w, 12.8% w/w, 12.9% w/w or 13% w/w.

In two preferred embodiments, also in combination with any of the above-described embodiments, the liquid suspension according to the first object of the invention comprises reparixin in:
- an amount between 2% w/w and 5.5% w/w, preferably between 3% w/w and 5.5% w/w, more preferably between 4% w/w and 5.5% w/w, more preferably between 4.5% w/w and 5.5% w/w, more preferably between 4.7% w/w and 5.3% w/w, more preferably between 4.9% w/w and 5.1% w/w, even more preferably in an amount of 5% w/w; this embodiment is suitable for administration to a pediatric subject, wherein the pediatric subject is less than 14 years of life; or
- an amount between 11.5% w/w and 12.5% w/w, preferably between 11.7% w/w and 12.3% w/w, even more preferably between 11.9% w/w and 12.1% w/w, most preferably in an amount of 12% w/w; this embodiment is suitable for administration to an adult subject, wherein the adult subject is more than 14 years of life.

In an embodiment, also in combination with any of the above-described embodiments, said antifoaming agent is a silicone derivative, preferably selected from cyclomethicone, dimethicone and simethicone, more preferably from dimethicone and simethicone.

The presence of the antifoaming agent in the liquid suspension is advantageous since it prevents and/or hinders the formation of foam on the surface of the suspension.

Preferably, said simethicone is in an amount between 0.003% w/w and 0.6% w/w, preferably between 0.003% w/w and 0.3% w/w, more preferably between 0.003% w/w and 0.009% w/w, even more preferably between 0.003% w/w and 0.007% w/w, most preferably in an amount of 0.005% w/w.

Preferably, said dimethicone is in an amount between 0.5% w/w and 5% w/w.

In an embodiment, also in combination with any of the above-described embodiments, said buffering agent is able to maintain the pH of the suspension at a value between 4 and 6, preferably between 4.5 and 5.5, more preferably at a value of 5. Preferably, said buffering agent is selected from citrate buffer and phosphate buffer, more preferably it is citrate buffer. Preferably, said citrate buffer comprises, preferably consists of, monohydrate citric acid in an amount between 0.06% w/w and 0.6% w/w, preferably between 0.06% w/w and 0.3% w/w, more preferably between 0.06% w/w and 0.1% w/w, even more preferably between 0.06% w/w and 0.07% w/w, most preferably in an amount of 0.064% w/w, and dihydrate sodium citrate in an amount between 0.2% w/w and 2% w/w, preferably between 0.2% w/w and 1% w/w, more preferably between 0.2% w/w and 0.3% w/w, most preferably in an amount of 0.208% w/w. Preferably, within the above ranges, the relative amount of monohydrate citric acid and dihydrate sodium citrate are selected such as to obtain a pH between 4 and 6, preferably between 4.5 and 5.5, more preferably at a value of 5.

In an embodiment, also in combination with any of the above-described embodiments, said preservative agent is selected from potassium sorbate, sodium benzoate, methyl parabens and propyl parabens. The presence of the preservative agent in the formulation is advantageous since it prevents or retards microbial growth.

Preferably, said potassium sorbate is in an amount between 0.1% w/w and 0.2% w/w, more preferably in an amount of 0.15% w/w.

Preferably, said sodium benzoate is in an amout between 0.02% w/w and 0.5% w/w. Preferably, said methyl parabens, propyl parabens or a combination thereof, are in an amount between 0.015% w/w and 0.2% w/w.

In an embodiment, also in combination with any of the above-described embodiments, said water insoluble polymer is selected from microcrystalline cellulose and crospovidone. The water insoluble polymer acts as anticaking agent and is used to prevent agglomeration of a particulate solid into lumps or cohesive cakes avoiding and/or limiting the formation of sediment.

Crospovidone, also known as polyvinylpolypyrrolidone, is a highly cross-linked modification of polyvinylpyrrolidone (PVP). In view of the cross-linking, crospovidone is insoluble in water.

In an embodiment, also in combination with any of the above-described embodiments, the liquid suspension according to the present invention further comprises an aqueous solvent, preferably water, in an amount up to 100% w/w.

In an embodiment, the liquid suspension according to the first object of the invention comprises, preferably consists of:
- reparixin in an amount between 2% w/w and 5.5% w/w, preferably between 3% w/w and 5.5% w/w, more preferably between 4% w/w and 5.5% w/w, more preferably between 4.5% w/w and 5.5% w/w, more preferably between 4.7% w/w and 5.3% w/w, more preferably between 4.9% w/w and 5.1% w/w, even more preferably in an amount of 5% w/w,
- xanthan gum in an amount between 0.15% w/w and 0.3% w/w, preferably between 0.18% w/w and 0.27% w/w, more preferably between 0.18% w/w and 0.23% w/w, even more preferably between 0.19% w/w and 0.21% w/w, most preferably in an amount of 0.2% w/w,
- sucrose in an amount between 25% w/w and 55% w/w, preferably between 27% w/w and 53% w/w, even more preferably between 29% w/w and 51% w/w, most preferably between 30% w/w and 50% w/w,
- polyvinylpyrrolidone, preferably polyvinylpyrrolidone K 30, in an amount between 1.5% w/w and 2.5% w/w, preferably between 1.7% w/w and 2.3% w/w, more preferably between 1.9% w/w and 2.1% w/w, even more preferably in an amount of 2% w/w,
- crospovidone in an amount between 2.25% w/w and 2.75% w/w, preferably in an amount of 2.5% w/w,
- optionally one or more of the following components: simethicone in an amount between 0.003% w/w and 0.006% w/w, preferably between 0.004% w/w and 0.006% w/w, more preferably in an amount of 0.005% w/w, monohydrate citric acid in an amount between 0.06% w/w and 0.6% w/w, preferably between 0.06% w/w and 0.3% w/w, more preferably between 0.06% w/w and 0.1% w/w, even more preferably between 0.06% w/w and 0.07% w/w, most preferably in an amount of 0.064% w/w, dihydrate sodium citrate in an amount between 0.2% w/w and 2% w/w, preferably between 0.2% w/w and 1% w/w, more preferably between 0.2% w/w and 0.3% w/w, most preferably in an amount of 0.208% w/w, potassium sorbate in an amount between 0.1% w/w and 0.2% w/w, preferably in an amount of 0.15% w/w,
- an aqueous solvent, preferably water, in an amount up to 100% w/w.

Preferably, within the above ranges, the relative amount of monohydrate citric acid and dihydrate sodium citrate are selected such as to obtain a pH between 4 and 6, preferably between 4.5 and 5.5, more preferably at a value of 5.

In an alternative embodiment, the liquid suspension according to the first object of the invention comprises, preferably consists of:
- reparixin in an amount between 11.5% w/w and 12.5% w/w, preferably between 11.7% w/w and 12.3% w/w, even more preferably between 11.9% w/w and 12.1% w/w, most preferably in an amount of 12% w/w,
- xanthan gum in an amount between 0.15% w/w and 0.3% w/w, preferably between 0.18% w/w and 0.27% w/w, more preferably between 0.18% w/w and 0.23% w/w, even more preferably between 0.19% w/w and 0.21% w/w, most preferably in an amount of 0.2% w/w,
- sucrose in an amount between 25% w/w and 55% w/w, preferably between 27% w/w and 53% w/w, even more preferably between 29% w/w and 51% w/w, most preferably between 30% w/w and 50% w/w,
- polyvinylpyrrolidone, preferably polyvinylpyrrolidone K 30, in an amount between 2.5% w/w and 3.5% w/w, preferably between 2.7% w/w and 3.3% w/w, more preferably between 2.9% w/w and 3.1% w/w, even more preferably in an amount of 3% w/w,
- crospovidone in an amount between 2.25% w/w and 2.75% w/w, preferably in an amount of 2.5% w/w,
- optionally one or more of the following components: simethicone in an amount between 0.003% w/w and 0.006% w/w, preferably between 0.004% w/w and 0.006% w/w, more preferably in an amount of 0.005% w/w, monohydrate citric acid in an amount between 0.06% w/w and 0.6% w/w, preferably between 0.06% w/w and 0.3% w/w, more preferably between 0.06% w/w and 0.1% w/w, even more preferably between 0.06% w/w and 0.07% w/w, most preferably in an amount of 0.064% w/w, dihydrate sodium citrate in an amount between 0.2% w/w and 2% w/w, preferably between 0.2% w/w and 1% w/w, more preferably between 0.2% w/w and 0.3% w/w, most preferably in an amount of 0.208% w/w, potassium sorbate in an amount between 0.1% w/w and 0.2% w/w, preferably in an amount of 0.15% w/w,
- an aqueous solvent, preferably water, in an amount up to 100% w/w.

Preferably, within the above ranges, the relative amount of monohydrate citric acid and dihydrate sodium citrate are selected such as to obtain a pH between 4 and 6, preferably between 4.5 and 5.5, more preferably at a value of 5.

In an embodiment, the liquid suspension according to the first object of the invention consists of:
- reparixin in an amount of 5% w/w,
- xanthan gum in an amount of 0.2% w/w,
- sucrose in an amount between 25% w/w and 55% w/w, preferably between 27% w/w and 53% w/w, even more preferably between 29% w/w and 51% w/w, most preferably between 30% w/w and 50% w/w,
- polyvinylpyrrolidone, preferably polyvinylpyrrolidone K 30, in an amount of 2% w/w,
- crospovidone in an amount of 2.5% w/w,
- simethicone in an amount of 0.005% w/w,
- monohydrate citric acid in an amount of 0.064% w/w,
- dihydrate sodium citrate in an amount of 0.208% w/w,
- potassium sorbate in an amount of 0.15% w/w.
- water up to 100%.

In another embodiment, the liquid suspension according to the first object of the invention consists of:
- reparixin in an amount of 12% w/w,
- xanthan gum in an amount of 0.2% w/w,
- sucrose in an amount between 25% w/w and 55% w/w, preferably between 27% w/w and 53% w/w, even more preferably between 29% w/w and 51% w/w, most preferably between 30% w/w and 50% w/w,
- polyvinylpyrrolidone, preferably polyvinylpyrrolidone K 30, in an amount of 3% w/w,
- crospovidone in an amount of 2.5% w/w,
- simethicone in an amount of 0.005% w/w,
- monohydrate citric acid in an amount of 0.064% w/w,
- dihydrate sodium citrate in an amount of 0.208% w/w,
- potassium sorbate in an amount of 0.15% w/w.
- water up to 100%.

In an embodiment, also in combination with any of the above-described embodiments, the amount of sucrose in the liquid suspension according to the first object of the invention is 30% w/w, 30.1% w/w, 30.2% w/w, 30.3% w/w, 30.4% w/w, 30.5% w/w, 30.6% w/w, 30.7% w/w, 30.8% w/w, 30.9% w/w, 31% w/w, 31.1% w/w, 31.2% w/w, 31.3% w/w, 31.4% w/w, 31.5% w/w, 31.6% w/w, 31.7% w/w, 31.8% w/w, 31.9% w/w, 32% w/w, 32.1% w/w, 32.2% w/w, 32.3% w/w, 32.4% w/w, 32.5% w/w, 32.6% w/w, 32.7% w/w, 32.8% w/w, 32.9% w/w, 33% w/w, 33.1% w/w, 33.2% w/w, 33.3% w/w, 33.4% w/w, 33.5% w/w, 33.6% w/w, 33.7% w/w, 33.8% w/w, 33.9% w/w, 34% w/w, 34.1% w/w, 34.2% w/w, 34.3% w/w, 34.4% w/w, 34.5% w/w, 34.6% w/w, 34.7% w/w, 34.8% w/w, 34.9% w/w, 35% w/w, 35.1% w/w, 35.2% w/w, 35.3% w/w, 35.4% w/w, 35.5% w/w, 35.6% w/w, 35.7% w/w, 35.8% w/w, 35.9% w/w, 36% w/w, 36.1% w/w, 36.2% w/w, 36.3% w/w, 36.4% w/w, 36.5% w/w, 36.6% w/w, 36.7% w/w, 36.8% w/w, 36.9% w/w, 37% w/w, 37.1% w/w, 37.2% w/w, 37.3% w/w, 37.4% w/w, 37.5% w/w, 37.6% w/w, 37.7% w/w, 37.8% w/w, 37.9% w/w, 38% w/w, 38.1% w/w, 38.2% w/w, 38.3% w/w, 38.4% w/w, 38.5% w/w, 38.6% w/w, 38.7% w/w, 38.8% w/w, 38.9% w/w, 39% w/w, 39.1% w/w, 39.2% w/w, 39.3% w/w, 39.4% w/w, 39.5% w/w, 39.6% w/w, 39.7% w/w, 39.8% w/w, 39.9% w/w, 40% w/w, 40.1% w/w, 40.2% w/w, 40.3% w/w, 40.4% w/w, 40.5% w/w, 40.6% w/w, 40.7% w/w, 40.8% w/w, 40.9% w/w, 41 % w/w, 41.1% w/w, 41.2% w/w, 41.3% w/w, 41.4% w/w, 41.5% w/w, 41.6% w/w, 41.7%w/w, 41.8% w/w, 41.9% w/w, 42% w/w, 42.1 % w/w, 42.2% w/w, 42.3% w/w, 42.4% w/w, 42.5% w/w, 42.6% w/w, 42.7% w/w, 42.8% w/w, 42.9% w/w, 43% w/w, 43.1% w/w, 43.2% w/w, 43.3% w/w, 43.4% w/w, 43.5% w/w, 43.6% w/w, 43.7% w/w, 43.8% w/w, 43.9% w/w, 44% w/w, 44.1% w/w, 44.2% w/w, 44.3% w/w, 44.4% w/w, 44.5% w/w, 44.6% w/w, 44.7% w/w, 44.8% w/w, 44.9% w/w, 45% w/w, 45.1% w/w, 45.2% w/w, 45.3% w/w, 45.4% w/w, 45.5% w/w, 45.6% w/w, 45.7% w/w, 45.8% w/w, 45.9% w/w, 46% w/w, 46.1% w/w, 46.2% w/w, 46.3% w/w, 46.4% w/w, 46.5% w/w, 46.6% w/w, 46.7% w/w, 46.8% w/w, 46.9% w/w, 47% w/w, 47.1% w/w, 47.2% w/w, 47.3% w/w, 47.4% w/w, 47.5% w/w, 47.6% w/w, 47.7% w/w, 47.8% w/w, 47.9% w/w, 48% w/w, 48.1% w/w, 48.2% w/w, 48.3% w/w, 48.4% w/w, 48.5% w/w, 48.6% w/w, 48.7% w/w, 48.8% w/w, 48.9% w/w, 49% w/w, 49.1% w/w, 49.2% w/w, 49.3% w/w, 49.4% w/w, 49.5% w/w, 49.6% w/w, 49.7% w/w, 49.8% w/w, 49.9% w/w or 50% w/w.

In an embodiment, also in combination with any of the above-described embodiments, the liquid suspension according to the first object of the invention has a zeta potential value between -3 mV and +3mV, preferably between -2.5 mV and +2.5 mV, wherein said zeta potential value is measured by laser Doppler electrophoresis using Zetasizer nano ZS (Malvern Instruments, Worcestershire, UK) according to manufacturer's instructions.

In an embodiment, also in combination with any of the above-described embodiments, the liquid suspension according to the first object of the invention has a yeald stress value of at least 8 Pa, wherein said yeald stress value is measured by Rheometer MCR 102 (Anton Paar, Graz, Austria) following the procedure described in Example 2 below.

In an embodiment, also in combination with any of the above embodiments, the liquid suspension according to the first object of the invention is stable for a period of at least one year, preferably for at least two years, meaning that during this period of time the API remains homogeneously suspended in the external phase ensuring the correct dose administration of the API or, in case a sediment is formed, it is easily resuspendable by shaking manually.

A second object of the invention is the liquid suspension according to any one of the above-described embodiments for use in the treatment of a disease or condition selected from psoriasis, rheumatoid arthritis, ulcerative colitis, acute respiratory distress syndrome (ARDS), idiopathic fibrosis and glomerulonephritis.

A further object of the invention is the liquid suspension according to any one of the above-described embodiments of the first object of the invention for use in the prevention of diabetes or in delaying the onset and the progression of diabetes, wherein said diabetes is preferably Type I diabetes.

A further object the present invention is the liquid suspension according to any one of the above-described embodiments of the first object of the invention for use in the treatment of an infection caused by SARS-CoV-2 or a variant thereof, preferably for use in the treatment of COVID-19, even more preferably for use in the improvement of respiratory function in subjects affected by pneumonia caused by SARS-CoV-2 or a variant thereof. In an embodiment, said improvement of respiratory function in said subjects means decreasing, delaying the onset of or preventing the need of supplemental oxygen requirement, mechanical ventilation use or admission to Intensive Care Unit.

As used herein, the term "pneumonia caused by SARS-CoV-2 or a variant thereof" indicates an inflammatory condition of the lung associated to an infection with SARS-CoV-2 or a variant thereof. This is a severe complication of COVID-19 affecting a percentage of the subjects.

In an embodiment, the liquid suspension according to any one of the above-described embodiments of the first object of the invention for use in the treatment of COVID-19 is administered in combination with a standard of care treatment for COVID-19 patients.

As used herein, by "standard of care treatment for COVID-19 patients" it is meant a pharmacological treatment that includes one or more drugs that have been approved by regulatory authorities i) as a treatment for COVID-19 or ii) as a treatment for other pathological conditions and symptoms thereof and that are used in clinical practice as a treatment of symptoms and complications that are associated to COVID-19. Preferably, said standard of care treatment consists in the treatment of the patient with at least one drug selected from: i) antiviral drugs, preferably remdesivir; ii) antipyretic, analgesic and antiinflammatory drugs, preferably non-steroidal anti inflammatory drugs, corticosteroids, cytokine, chemokine and interleukin inhibitors, more preferably selected from dexhamethasone, paracetamol, anakinra, celecoxib, prednisone, prednisolone, methylprednisolone, piryrone, and tramadol; iii) antibiotics, preferably selected from piperacillin in association with tazobactam, azithromycin and ceftriazone; and iv) anticoagulants and antithrombotic drugs, preferably selected from enoxaparin and acetylsalicylic acid.

Preferably, said subjects affected by pneumonia caused by SARS-CoV-2 or a variant thereof have lymphocytopenia.

According to a preferred embodiment, said subjects with lymphocytopenia are adult patients (14 years and above) and have blood concentration of lymphocytes below 1000, below 900, below 850, below 800 or below 750 cells/microliter.

According to another preferred embodiment, said patients with lymphocytopenia are pediatric patients (below 14 years of age) and have blood concentration of lymphocytes below 3000, below 2800, below 2500, below 2300 or below 2000 cells/microliter.

A further object of the present invention is the liquid suspension according to any one of the above-described embodiments of the first object of the invention for use in the prevention or treatment of seizures in an individual.

According to a preferred embodiment, said seizures are acute symptomatic seizures (ASS).

According to another preferred embodiment, said seizures are associated to an established epilepsy (Established Epilepsy Seizures EES).

In an embodiment, also in combination with any of the above-described embodiments, the liquid suspension for use according to the present invention is a ready-to-use liquid suspension, namely a suspension already distributed through a liquid vehicle. Said liquid suspension can be single-dose, that is, intended for a single administration, or multidose, that is, intended for more than one administration.

In an embodiment, also in combination with any of the above-described embodiments, the liquid suspension for use according to the present invention is administered orally to a subject, preferably a mammal, even more preferably a human.

The invention will be further described in the following examples, which do not limit the scope of the invention as defined in the claims.

### EXAMPLES

All the percentages indicated in the following tables are to be intended as %w/w.

### Example 1 - Preparation of suspensions comprising reparixin

Several suspensions comprising reparixin were prepared. All the suspensions contained at least the following excipients:
- a viscosity agent, which regulates the viscosity of the composition to achieve optimal rheological properties;
- a wetting agent, which is necessary to increase the wettability of reparixin in water, which is per se low;
- a sweetener, which is used to mask the unpleasant taste of reparixin;
- an antifoaming agent, which is used to prevent and/or hinder the formation of foam on the surface of the suspension;
- a preservative agent, which is used for preventing or retarding microbial growth.
- a buffering agent, which is used for maintaining the pH of the suspension at a value between 4 and 6, preferably between 4.5 and 5.5, more preferably 5.

The materials used for preparing the suspensions are indicated in Table 1.

**Table 1**

| **Trade Name** | **Chemical Name** | **Supplier** |
|---|---|---|
| Sucrose Compri O | Sucrose | Südzucker |
| Sucralose | Sucralose | Merck-Millipore |
| Potassium Sorbate | Potassium Sorbate | Merck-Millipore |
| Avicel CL-611 | Microcrystalline cellulose + Sodium Carboxymethylcellulose | Dupont |
| Xantural 75 | Xanthan Gum | Kelco |
| Kolliphor SLS | Sodium Lauryl Sulphate | Basf |
| Kolliphor P188 (poloxamer 188) | polyoxyethylene-polyoxypropylene copolymer average MW 7608-9510 | Basf |
| Kollidon 30 | Polyvinyl Pyrrolidone | Basf |
| Kolliphor P407 (poloxamer 407) | polyoxyethylene-polyoxypropylene copolymer average MW 9840-14600 | Basf |
| Simethicone | Silicone Antifoam | Wacker |
| Monohydrate Citric Acid | Monohydrate Citric Acid | Merck-Millipore |
| Dihydrate Sodium Citrate | Dihydrate Sodium Citrate | Sigma-Aldrich |
| Kollidon CL-M (crospovidone) | 1-Ethenyl-2pyrrolidinone homopolymer | Basf |

The type and/or amount of the following components were varied among the suspensions:
- the viscosity agent. In particular, xanthan gum and Avicel CL-611 were tested as viscosity agents. Avicel CL-611 is a spray-dried blend of microcrystalline cellulose and sodium carboxymethylcellulose, wherein sodium carboxymethylcellulose is present in an amount between 11.3% w/w and 18.8% w/w;
- the wetting agent. In particular, sodium lauryl sulfate (SLS), poloxamer 407, poloxamer 188 and polyvinylpyrrolidone (PVP) were tested as wetting agents. Poloxamer 407 was immediately rejected due to its insufficient ability to disperse reparixin;
- the sweetener. In particular, sucrose and sucralose were tested as sweeteners. Sucrose has a high glycemic index, while sucralose is an artificial sweetener with zero glycemic index.

The procedure used for the preparation of the suspensions is described in the following. The selected amounts of antifoaming agent, wetting agent, preservative agent and buffering agents were added to 40 g of distilled water, and the mixture was maintained under magnetic stirrer until a solution was obtained. Then, the selected amount of reparixin was added and dispersed. Once the API was homogenously suspended, the selected amounts of other excipients (viscosity agent, sweetener and anticaking agent, when present) were added and dissolved. Finally, water was added up to a final weight of 100 g.

In the following, the composition of the prepared liquid suspensions will be reported, while the respective characterization data will be shown in Examples 2 and 3.

Table 2 below reports the liquid suspensions prepared by using SLS as wetting agent. Specifically, the prepared suspensions contained 0.11% w/w SLS, 40% w/w sucrose as sweetener, 0.15% w/w potassium sorbate as preservative agent, monohydrate citric acid and dihydrate sodium citrate as buffering agents, 0.005% w/w simethicone as antifoaming agent and water as solvent.

The type/amount of the viscosity agent used (xanthan gum or Avicel CL-611) and the amounts of buffering agent were varied among the prepared suspensions.

**Table 2**

| **Suspension** | **41** | **55** | **56** | **57** | **58** | **59** |
|---|---|---|---|---|---|---|
| **Reparixin** | 5.00% | 5.00% | 5.00% | 5.00% | 5.00% | 5.00% |
| **Sucrose** | 40.00% | 40.00% | 40.00% | 40.00% | 40.00% | 40.00% |
| **Potassium sorbate** | 0.15% | 0.15% | 0.15% | 0.15% | 0.15% | 0.15% |
| **Viscosity agent** | Avicel CL-611 1.00% | Xanthan gum 0.10% | Xanthan gum 0.25% | Avicel CL-611 1.00% | Avicel CL-611 1.00% | Avicel CL-611 1.00% |
| **Monohydrate citric acid** | 0.064% | 0.064% | 0.064% | 0.320% | 0.640% | 1.240% |
| **Dihydrate sodium citrate** | 0.208% | 0.208% | 0.208% | 1.040% | 2.080% | 4.140% |
| **Simethicone** | 0.005% | 0.005% | 0.005% | 0.005% | 0.005% | 0.005% |
| **SLS** | 0.11% | 0.11% | 0.11% | 0.11% | 0.11% | 0.11 % |
| **Water** | Up to 100% | Up to 100% | Up to 100% | Up to 100% | Up to 100% | Up to 100% |

Table 3 below reports the liquid suspensions prepared by using poloxamer 188 as wetting agent in combination with xanthan gum as viscosity agent. The suspensions contained 0.15% w/w potassium sorbate as preservative agent, 0.064% w/w monohydrate citric acid and 0.208% w/w dihydrate sodium citrate as buffering agents, 0.005% w/w simethicone as antifoaming agent and water as solvent.

The amounts of poloxamer 188 and xanthan gum were varied among the prepared suspensions, as well as the type/amount of sweetener (sucrose or sucralose).

**Table 3**

| **Suspension** | **73** | **74** | **82** | **83** | **84** | **89** |
|---|---|---|---|---|---|---|
| **Reparixin** | 5.00% | 5.00% | 5.00% | 5.00% | 5.00% | 5.00% |
| **Sweetener** | Sucrose 40.00% | Sucrose 40.00% | Sucrose 40.00% | Sucralose 0.06% | / | Sucrose 20.00% |
| **Potassium sorbate** | 0.15% | 0.15% | 0.15% | 0.15% | 0.15% | 0.15% |
| **Xanthan gum** | 0.20% | 0.40% | 0.40% | 0.40% | 0.40% | 0.40% |
| **Monohydrate citric acid** | 0.064% | 0.064% | 0.064% | 0.064% | 0.064% | 0.064% |
| **Dihydrate sodium citrate** | 0.208% | 0.208% | 0.208% | 0.208% | 0.208% | 0.208% |
| **Simethicone** | 0.005% | 0.005% | 0.005% | 0.005% | 0.005% | 0.005% |
| **Poloxamer 188** | 0.25% | 0.25% | 0.50% | 0.50% | 0.50% | 0.50% |
| **Water** | Up to 100% | Up to 100% | Up to 100% | Up to 100% | Up to 100% | Up to 100% |

Table 4 below reports the liquid suspensions prepared by using poloxamer 188 as wetting agent in combination with Avicel CL-611 as viscosity agent. The suspensions contained 0.25% poloxamer 188, 0.15% w/w potassium sorbate as preservative agent, 40% w/w sucrose as sweetener, 0.005% w/w simethicone as antifoaming agent and water as solvent.

The amounts of Avicel CL-611 and the type/amount of buffering agent (monohydrate citric acid/dihydrate sodium citrate or monohydrate citric acid/NaOH 1M) were varied among the different suspensions prepared.

In addition to the suspensions reported in Table 4, also suspension 80, having a composition corresponding to suspension **79** but containing 1.00% w/w Blanose (sodium carboxymethylcellulose) instead of 3.00% w/w Avicel CL-611, was prepared.

**Table 4**

| **Suspension** | **68** | **69** | **70** | **71** | **72** | **78** | **79** |
|---|---|---|---|---|---|---|---|
| **Reparixin** | 5.00% | 5.00% | 5.00% | 5.00% | 5.00% | 5.00% | 5.00% |
| **Sucrose** | 40.00% | 40.00% | 40.00% | 40.00% | 40.00% | 40.00% | 40.00% |
| **Potassium sorbate** | 0.15% | 0.15% | 0.15% | 0.15% | 0.15% | 0.15% | 0.15% |
| **Avicel CL-611** | 1.00% | 1.00% | 1.00% | 1.00% | 1.00% | 2.00% | 3.00% |
| **Monohydrate citric acid** | 0.320% | 0.10% | 0.40% | 0.40% | 0.40% | 0.064% | 0.064% |
| **Other buffering agent** | Dihydrate sodium citrate 1.040% | NaOH 1M 0.03% | NaOH 1M 0.06% | NaOH 1M 0.126% | NaOH 1M 0.162% | Dihydrate sodium citrate 0.208% | Dihydrate sodium citrate 0.208% |
| **Simethicone** | 0.005% | 0.005% | 0.005% | 0.005% | 0.005% | 0.005% | 0.005% |
| **Poloxamer 188** | 0.25% | 0.25% | 0.25% | 0.25% | 0.25% | 0.25% | 0.25% |
| **Water** | Up to 100% | Up to 100% | Up to 100% | Up to 100% | Up to 100% | Up to 100% | Up to 100% |

Table 5 below reports the liquid suspensions prepared using polyvinylpyrrolidone as wetting agent in combination with xanthan gum as viscosity agent. Specifically, the prepared suspensions contained 2.00% w/w PVP K 30 as wetting agent, xanthan gum, 0.15% w/w potassium sorbate as preservative agent, 40% w/w sucrose as sweetener, 0.005% w/w simethicone as antifoaming agent, 0.064% w/w monohydrate citric acid and 0.208% w/w dihydrate sodium citrate as buffering agents, and water as solvent.

The amounts of xanthan gum were varied among the suspensions, as well as the amounts of crospovidone, used as anticaking agent (from 0% w/w to 2.50% w/w).

**Table 5**

| **Suspension** | **91** | **93** | **98** | **105** | **106** | **107** |
|---|---|---|---|---|---|---|
| **Reparixin** | 5.00% | 5.00% | 5.00% | 5.00% | 5.00% | 5.00% |
| **Sucrose** | 40.00% | 40.00% | 40.00% | 40.00% | 40.00% | 40.00% |
| **Potassium sorbate** | 0.15% | 0.15% | 0.15% | 0.15% | 0.15% | 0.15% |
| **Xanthan gum** | 0.40% | 0.40% | 0.20% | 0.20% | 0.20% | 0.20% |
| **Monohydrate citric acid** | 0.064% | 0.064% | 0.064% | 0.064% | 0.064% | 0.064% |
| **Dihydrate sodium citrate** | 0.208% | 0.208% | 0.208% | 0.208% | 0.208% | 0.208% |
| **Simethicone** | 0.005% | 0.005% | 0.005% | 0.005% | 0.005% | 0.005% |
| **PVP K 30** | 2.00% | 2.00% | 2.00% | 2.00% | 2.00% | 2.00% |
| **Crospovidone** | / | 2.50% | 2.50% | 0.50% | 1.50% | 2.00% |
| **Water** | Up to 100% | Up to 100% | Up to 100% | Up to 100% | Up to 100% | Up to 100% |

Table 6 reports the liquid suspensions prepared using polyvinylpyrrolidone as wetting agent in combination with Avicel-CL611 as viscosity agent. Specifically, the prepared suspensions contained 2.00% w/w PVP K30, Avicel CL-611, 0.15% w/w potassium sorbate as preservative agent, 40% w/w sucrose as sweetener, 0.005% w/w simethicone as antifoaming agent, 0.064% w/w monohydrate citric acid and 0.208% w/w dihydrate sodium citrate as buffering agents, and water as solvent.

The amounts of Avicel CL-611 and the amounts of crospovidone, used as anticaking agent (from 0% w/w to 1% w/w) were varied among the prepared suspensions.

**Table 6**

| **Suspension** | **99** | **104** | **108** |
|---|---|---|---|
| **Reparixin** | 5.00% | 5.00% | 5.00% |
| **Sucrose** | 40.00% | 40.00% | 40.00% |
| **Potassium sorbate** | 0.15% | 0.15% | 0.15% |
| **Avicel CL-611** | 1.00% | 1.00% | 2.00% |
| **Monohydrate citric acid** | 0.064% | 0.064% | 0.064% |
| **Dihydrate sodium citrate** | 0.208% | 0.208% | 0.208% |
| **Simethicone** | 0.005% | 0.005% | 0.005% |
| **PVP K 30** | 2.00% | 2.00% | 2.00% |
| **Crospovidone** | / | 1.00% | / |
| **Water** | Up to 100 | Up to 100 | Up to 100 |

Finally, the impact of the type and amount of sweetener included in suspensions containing PVP as wetting agent and crospovidone as anticaking agent was further investigated.

Table 7 reports the liquid suspensions prepared using 2.00% w/w PVP K 30, 2.50% w/w crospovidone, 0.20% w/w xanthan gum as viscosity agent, 0.15% w/w potassium sorbate as preservative agent, 0.005% w/w simethicone as antifoaming agent, 0.064% w/w monohydrate citric acid and 0.208% w/w dihydrate sodium citrate as buffering agents, and water as solvent.

The type/amount of sweetener (sucrose or sucralose) was varied among the suspensions prepared.

**Table 7**

| **Suspension** | **165** | **159** | **160** | **166** | **168** |
|---|---|---|---|---|---|
| **Reparixin** | 5.00% | 5.00% | 5.00% | 5.00% | 5.00% |
| **Sweetener** | Sucrose 20.00% | Sucrose 30.00% | Sucrose 50.00% | Sucrose 60.00% | Sucralose 0.60% |
| **Potassium sorbate** | 0.15% | 0.15% | 0.15% | 0.15% | 0.15% |
| **Xanthan gum** | 0.20% | 0.20% | 0.20% | 0.20% | 0.20% |
| **Monohydrate citric acid** | 0.064% | 0.064% | 0.064% | 0.064% | 0.064% |
| **Dihydrate sodium citrate** | 0.208% | 0.208% | 0.208% | 0.208% | 0.208% |
| **Simethicone** | 0.005% | 0.005% | 0.005% | 0.005% | 0.005% |
| **PVP K30** | 2.00% | 2.00% | 2.00% | 2.00% | 2.00% |
| **Crospovidone** | 2.50% | 2.50% | 2.50% | 2.50% | 2.50% |
| **Water** | Up to 100% | Up to 100% | Up to 100% | Up to 100% | Up to 100% |

### Example 2 - Determination of zeta potential and rheological properties

The zeta potential values and the rheological properties of the liquid suspensions reported in Example 1 were determined in order to evaluate their stability.

Reparixin is an insoluble active pharmaceutical ingredient that has large particle size and produces coarse suspensions. Because of this large particle size, the level of stability of the suspension is determined by gravitational forces, rather than by electrostatic interactions, which are insufficient to provide stability and kinetic stabilization. To ensure stability of suspensions containing reparixin, the zeta potential value has to be equal or near zero, and the particles of the internal phase must be surrounded by a hydration layer which hides their charge and ensures that, even when a sediment is formed, it is a loose sediment which is easily resuspendable, instead of a compact sediment difficult to resuspend. The increase of zeta potential (in terms of absolute value) corresponds to a condition where this hydration layer surrounding the particles of the internal phase is less present or absent, leading to an increase of the sedimentation rate.

As regards the rheological properties, yield stress and thixotropy of the prepared suspensions were determined to gain information about the physical stability of the suspensions. The yield stress (τ0) is a material property that can be defined as the stress corresponding to the yield point at which the material begins to flow or deform plastically. A suspension is considered stable if the yield stress is higher than the sedimentation stress (τS), which can be defined as the stress a particle performs on the liquid. If τ0 is higher than τS, sedimentation will not occur, and the suspension can be considered stable. Generally, a yield stress around 8 or more is considered a good value against sedimentation for ready-to-use liquid suspensions as final formulations.

The physical stability of a suspension is also evaluated based on the thixotropy hysteresis area. Thixotropy is an imperative property of the polymer system selected as viscosity agent. Indeed, the consistency (e.g. viscosity) of the external phase, caused by suspending agent, should decrease when a shear stress, such as the manual shaking, is applied so as to guarantee the re-dispersibility of sediment. In scientific literature, the thixotropy area is usually correlated with the suspension stability. As demonstrated by E. C. Foernzler, the sedimentation velocity is directly proportional to the reciprocal of the hysteresis area, which means that the physical stability is directly proportional to thixotropy (Ernest C. Foernzler et al., J. Am. Pharm. Assoc. 49 (1960) 249-252).

Based on the above, the produced suspensions were characterized in terms of zeta-potential value, rheological properties and capability of re-dispersion.

The values of zeta potential of the prepared suspensions were measured by laser Doppler electrophoresis using Zetasizer nano ZS (Malvern Instruments, Worcestershire, UK) according to manufacturer's instructions.

Rheological properties such as yield stress and thixotropy (hysteresis) were evaluated through Rheometer MCR 102 (Anton Paar, Graz, Austria).

The determination of yield stress was performed increasing linearly the shear rate stepwise between 0.1 and 100 s⁻¹ to generate a flow curve (shear stress vs. shear rate). Then, the resulting flow curve was fitted with a Herschel-Bulkley regression model, and the yield point was determined by extrapolating the flow curve to zero shear rate.

The thixotropy of the suspensions was evaluated via hysteresis. This measurement was carried out using an initial pre-shear at 5 1/s, the up-flow curve was then generated increasing the shear rate from 5 to 131 s⁻¹ (log scale) and the down-flow curve was obtained decreasing the shear rate from 131 to 5 s⁻¹. The area between the up-curve and down-curve was calculated and it is correlated with the thixotropy of the system. All analyses were carried out at room temperature using a 25 mm measuring plate and setting-up the gap at 1 mm.

If sediment was present, the capability of re-dispersion was evaluted considering the number of 180° rotations allowing the re-dispersion of sediment.

Table 8 below reports the values of zeta potential and rheological properties obtained for the suspensions reported in Table 2 above.

**Table 8**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **41** | -13.63 | 0.00 | 281.31 | > 30 (Sediment was redispersed only after vigorous manual shaking) |
| **55** | -21.3 | 1.64 | 190.95 | 10 |
| **56** | -17.6 | 1.81 | 84.18 | 5 |
| **57** | -13.5 | 0.19 | 43.39 | 3 |
| **58** | -10.57 | 0.68 | 26.172 | 3 |
| **59** | -7.46 | 0.00 | 2.26 | 2 |

As can be seen, the zeta potential values of the suspensions with 0.11% w/w SLS as wetting agent were all too high in terms of absolute value to allow stability of the suspensions. The increase of the concentration of citrate buffer (dihydrate sodium citrate + monohydrate citric acid) slightly diminished the absolute value of the zeta-potential, which however was still too high. Based on these results, SLS was ruled out as wetting agent due to its inability to produce stable suspensions of reparixin.

Table 9 below reports the values of zeta potential and rheological properties obtained for the suspensions reported in reported in Table 3 above.

**Table 9**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **73** | -0.43 | 1.06 | 104.96 | 20 (vigorous manual shaking is needed to resuspend the sediment |
| **74** | -0.23 | 14.30 | 1376.90 | No sediment |
| **82** | -0.5 | 14.00 | 760.6 | No sediment |
| **83** | -21.2 | 2.56 | 153.9 | 20 |
| **84** | -15.96 | NA | NA | 30 |
| **89** | -3.37 | NA | NA | 10^{§} |

As can be seen, suspensions **73, 74** and **82,** containing poloxamer 188 and 40% w/w sucrose, had zeta potential values near zero. However, when the percentage of sucrose was decreased (suspensions **84,** without sucrose, and **89,** with 20.00% w/w sucrose) or when sucrose was substituted with sucralose (suspension **83**) the absolute value of the zeta potential increased.

This trend was indicative of a surprising impact of the type and amount of sweetener included in the formulation on the zeta potential value of the suspension and, consequently, on its stability. As demonstrated, the amount of sucrose significantly impacted on zeta potential and rheological properties. Indeed, the increase of sucrose amount (from 0% w/w to 40% w/w) caused a decrease of the zeta- potential, in terms of absolute value.

Without being bound to a theory or principle, this trend can be explained by an unexpected formation of a hydration layer of sucrose surrounding the suspended particles which allows to increase the distance between the ions (cations and anions) decreasing, in this way, the absolute value of zeta potential. This hydration layer ensures that, even in case a sediment is formed, this sediment is easily resuspendable by manual shaking, since it is loose and not compact. At the same time, the increase of sucrose concentration produced an increase of both yield stress and hysteresis area, suggesting an improvement of the suspension stability.

With regard to the rheological properties, suspensions **74** and **82** exhibited the best values in terms of yeald stress and hysteresis relative area.

Even though suspension **74** exhibited a zeta-potential value near zero and had suitable values of yield stress and hysteresis area, it was rejected because of the presence of suspended aggregates which persisted over the time. Suspension **82** was further characterized by frequency sweep analysis, as described in Example 3.

Table 10 below reports the values of zeta potential and rheological properties obtained for the suspensions reported in Table 4 avove and of suspension **80** described above.

**Table 10**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **68** | -1.30 | 0.84 | 48.173 | >20 (vigorous manual shaking is needed to resuspend the sediment |
| **69** | -1.60 | <0.01 | 3.20 | 20 |
| **70** | -1.10 | <0.01 | 45.91 | >20 (vigorous manual shaking is needed to resuspend the sediment |
| **71** | -1.10 | 0.40 | 86.88 | 10 |
| **72** | -0.96 | 0.00 | 21.70 | 8 |
| **78** | -1.26 | 5.09 | 99.69 | NA |
| **79** | -1.6 | 5.88 | 1205.70 | NA |
| **80** | -0.83 | 3.04 | 46.79 | Not-redispersible sediment |

As can be seen, all the suspensions of Table 4 exhibited zeta potential values near zero, confirming that the use of 40% sucrose as sweetener allowed to achieve this trend. However, all the suspensions were characterized by too low values of yield stress for a ready-to-use liquid suspension as final formulation. Based on these results, the use of Avicel CL-611 as viscosity agent was ruled out. The use of this viscosity agent produced suspension with non-adequate rheological properties in terms of yield stress and hysteresis loop (thixothropy).

Table 11 below reports the values of zeta potential and rheological properties obtained for the suspensions reported in Table 5 above.

**Table 11**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **91** | -2.37 | 8.92 | 951.16 | NA (No sediment-Presence of suspended aggregate) |
| **93** | -0.13 | 20.06 | 894.08 | NA (No sediment - The dispersion was too viscous) |
| **98** | -0.60 | 10.25 | 612.760 | NA (No sediment) |
| **105** | -0.66 | 2.30 | 62.70 | NA (No sediment) |
| **106** | -1.03 | 8.82 | 109.54 | NA (No sediment) |
| **107** | -0.53 | 8.45 | 158.2 | NA (No sediment) |

As can be seen, when 40% sucrose was used as sweetener with PVP K 30 as wetting agent, suspensions characterized by zeta-potential values near zero were obtained, thus confirming the trend of the results reported above with respect to the role of sucrose in stabilizing the zeta potential values of the suspensions. The addition of crospovidone as anticaking agent improved the rheological properties of the suspensions, particularly at high percentages (suspensions **93** and **98,** containing 2.50% w/w of crospovidone). When 40% w/w xanthan gum was used as viscosity agent, the suspension was too viscous and thus difficult to handle (suspension **93**). When the amount of xanthan gum was reduced to 0.20% w/w, this issue was solved and the suspension was easy to handle.

Table 12 below reports the values of zeta potential and rheological properties obtained for the suspensions reported in Table 6 above.

**Table 12**

| **Suspension** | **Zeta-Potential (mV)** | **Yield stress (Pa)** | **Hysteresis Relative Area (\|Pa/s\|)** | **Re-dispersion after 1 month of storage (number of 180° rotation)** |
|---|---|---|---|---|
| **99** | -0.30 | 4.14 | 1542.2 | 5 (Persistent presence of floating foam at t0 and after 1 month as well) |
| **104** | This suspension was not characterized due to persistent presence of a floating foam (Reparixin was not completable wetted) | | | |
| **108** | -1.30 | 0.23 | 40.97 | NA (Persistent floating foam) |

As can be seen, the use of Avicel CL-611 as viscosity agent in combination with PVP K 30 as wetting agent resulted in suspensions having unsuitable rheological properties.

Table 13 below reports the values of zeta potential and rheological properties obtained for the suspensions reported in Table 7 above.

**Table 13**

| **Suspension** | **Zeta-Potential (mV)** | **Yield stress (Pa)** | **Hysteresis Relative Area (\|Pa/s\|)** | **Appereance** |
|---|---|---|---|---|
| **165** | -7.42 | 1.48 | 36.057 | Omogeneous appearance, presence of foam at the surface |
| **159** | -2.58 | 11.70 | 395.70 | Omogeneous appearance |
| **160** | -1.26 | 8.72 | 2519.4 | Omogeneous appearance |
| **166** | -0.068 | 0.00 | 4791.7 | Omogeneous appearance, presence of a persistent layer of foam at the surface |
| **168** | -5.77 | 0.43 | 23.447 | Omogeneous appearance, presence of a persistent layer of foam at the surface |

This experiment was carried out to further investigate and confirm the impact of the type/amount of sweetener selected in the stabilization of zeta potential values of the suspensions. As can be seen, when 20% w/w sucrose was used as sweetener (suspension **165**), the absolute value of the zeta potential was too high and the yeald stress value was too low, meaning that the produced suspension was not stable. The same trend was observed when 0.60% sucralose was used as sweetener (suspension **168**).

When 60% w/w was used as sweetener (suspension **166**), the presence of a persistent layer of foam at the surface was observed.

When sucrose 30% w/w and 50% w/w was used as sweetener (suspensions **159** and **160**, respectively) suspensions with zeta potential values near zero and suitable rheological properties were produced.

These results confirmed the unexpected impact of the type and amount of sweetener added in the suspension to mask the unpleasant taste of reparixin on the values of zeta potential and rheological properties and, consequently, on the stability of the suspension. In particular, based on these results, sucralose and the amounts of 20% w/w and 60% w/w of sucrose were ruled out to produce stable liquid suspensions of reparixin.

### Example 3 - Frequency sweep analysis

Frequency sweep analysis is employed for the prediction of the long-term physical stability of suspensions. Precisely, frequency sweep is used for describing the time-dependent behavior of suspensions in the non-destructive deformation range (e.g. linear viscoelastic region previously determined through an amplitude analysis). High frequencies are used to simulate fast motion on short timescales, while low frequencies simulate slow motion on long timescales. The storage modulus can be used as a measure of the elastic component (solid-like) of the sample while the loss modulus is considered as the viscous component (liquid-like) of the sample. Whichever modulus is dominant at a particular frequency will indicate whether the fully structured material appears to be elastic or viscous. When the storage modulus is higher than the loss modulus, suspension has more elastic than viscous meaning that they are characterized by a solid-like behavior and, in this condition, sedimentation is unlikely to occur (stability of suspension). On the other hand, when the viscous modulus is higher than the elastic one, sedimentation may occur.

Frequency sweep analysis was performed using the Rheometer MCR 102 (Anton Paar, Graz, Austria). Frequency sweep analysis was performed at decreasing angular frequency (from 62.8 to 0.0628 rad/s corresponding to 10-0.01 Hz) and constant strain, which was selected in the LVER (linear viscoelastic region) through oscillatory measurements. All analyses were performed at room temperature using a 25 mm measuring plate and setting-up the gap at 1 mm.

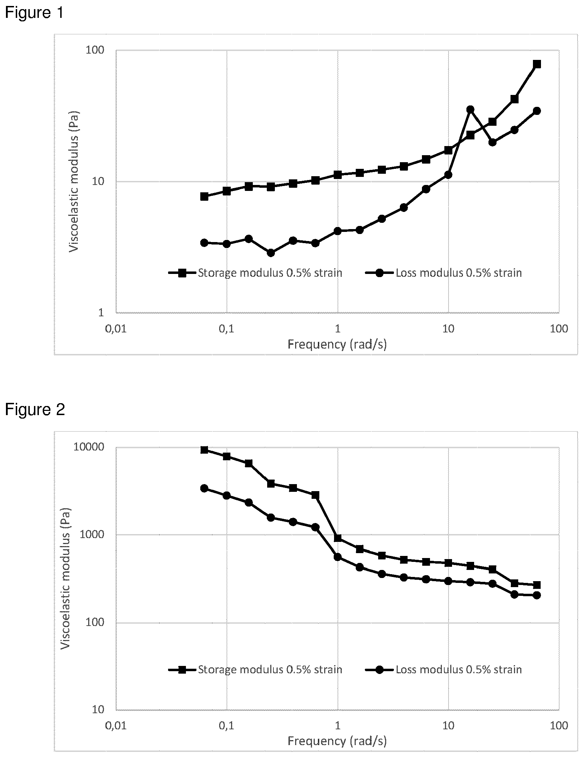

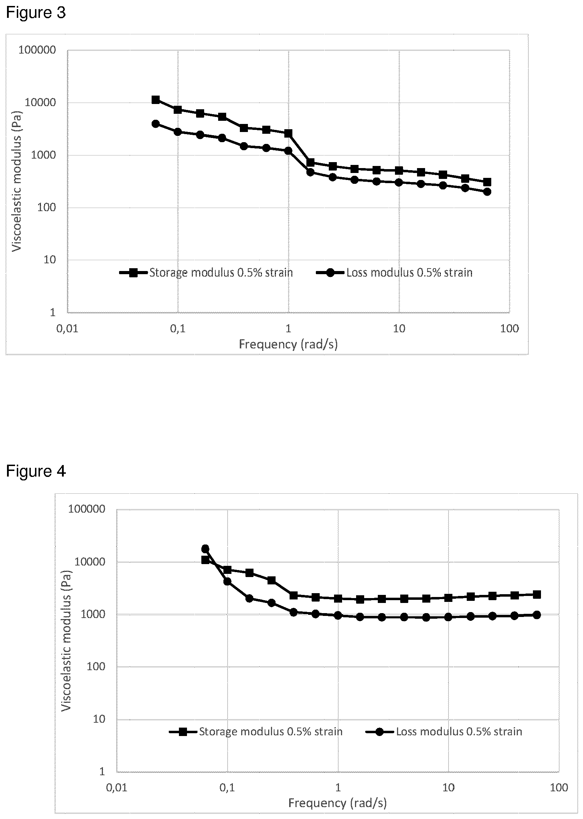

Figures 2 (suspension **98**) and Figure 3 (suspension **105**) show frequency sweep profiles of systems in which the storage modulus is higher than the loss modulus, suggesting that these suspensions are stable over time. In addition, both suspensions are characterized by a storage modulus value recorded at low frequency (around 0.1 rad/s) higher than 10 Pa, meaning that a possible dispersion stability can be assumed (T. G. Mezger, "The Rheology Handbook", 2nd Ed., Vincentz Network, Hannover, 2006). Figure 2 and Figure 3 show an increase of both loss and storage moduli at decreasing frequency values meaning that the corrisponding suspensions could become more structured and packed over the time.

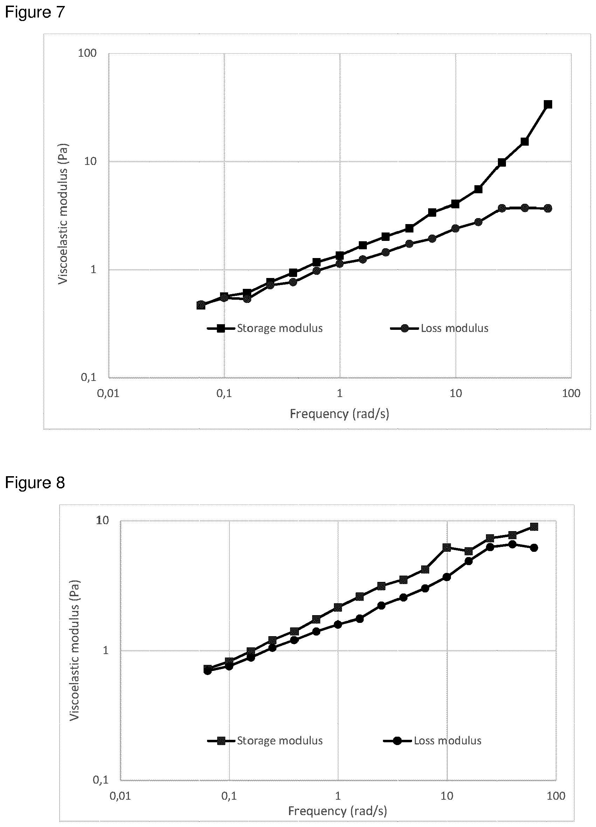

With regard to suspension **105**, despite having a good profile of frequency sweep, it was characterized by low values of yield stress and hysteresis area (Table 11 above). Figure 7 (suspension **159**) and Figure 8 (suspension **160**) show profiles associated to system in which the storage modulus is dominant over the loss modulus suggesting a stability of the suspension, even though at low frequency value the difference between the loss and the storage modulus becomes lower.

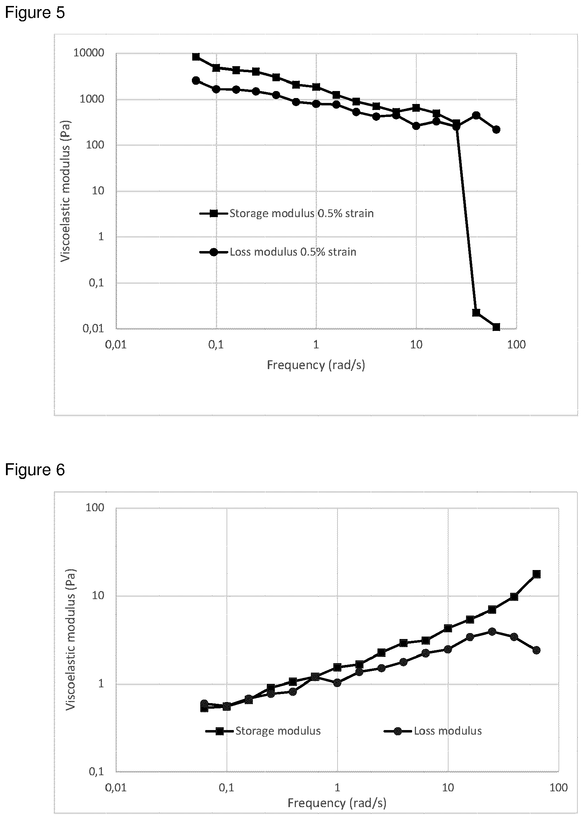

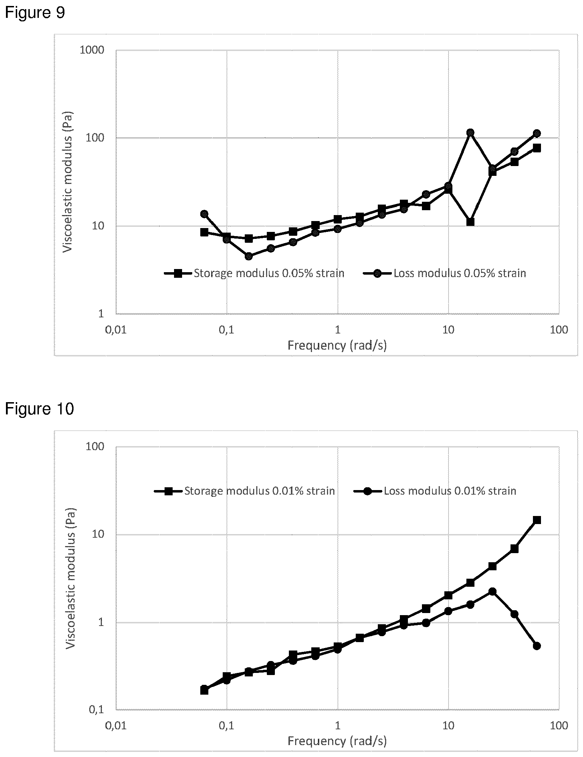

Figure 1 (suspension **82**), Figure 5 (suspension **107**) and Figure 9 (suspension **166**) are all associated to systems in which at high frequency values (between 10 and 100 rad/s) the loss modulus is higher than the storage modulus, which is indicative of a possible instability of suspension that can occur during short time storage.

Figure 4, 6 and 10 (suspensions **106, 165** and **168**) are associated with systems in which, at low frequency values, the loss modulus is higher than the storage modulus indicating a possible sedimentation phenomenon over the time (during long storage).

## Claims

1. A liquid suspension comprising:
- reparixin in an amount between 2% w/w and 13% w/w, preferably between 4% w/w and 13% w/w,
- xanthan gum in an amount between 0.15% w/w and 0.3% w/w,
- sucrose in an amount between 25% w/w and 55% w/w,
- polyvinylpyrrolidone in an amount between 1.8% w/w and 3.2% w/w,
- a water insoluble polymer in an amount between 2.25% w/w and 2.75% w/w.

2. The liquid suspension according to claim 1, wherein said water insoluble polymer is selected from microcrystalline cellulose and crospovidone.

3. The liquid suspension according to claim 1 or claim 2, wherein said water insoluble polymer is crospovidone, preferably in an amount of 2.5% w/w.

4. The liquid suspension according to any one of the preceding claims, further comprising an antifoaming agent, preferably a silicone derivative.

5. The liquid suspension according to claim 4, wherein said antifoaming agent is selected from:
- dimethicone in an amount between 0.5% w/w and 5% w/w, and
- simethicone in an amount between 0.003% w/w and 0.006% w/w, preferably in an amount of 0.005% w/w.

6. The liquid suspension according to any one of the preceding claims, further comprising a buffering agent, preferably selected from citrate buffer and phosphate buffer.

7. The liquid suspension according to claim 6, wherein said buffer is citrate buffer comprising monohydrate citric acid in an amount between 0.06% w/w and 0.6% w/w, preferably in an amount of 0.064% w/w, and dihydrate sodium citrate in an amount between 0.2% w/w and 0.3% w/w, preferably in an amount of 0.208% w/w.

8. The liquid suspension according to any one of the preceding claims, further comprising a preservative agent, preferably selected from potassium sorbate, sodium benzoate, methyl parabens and propyl parabens.

9. The liquid suspension according to claim 8, wherein said preservative agent is potassium sorbate in an amount between 0.1% w/w and 0.2% w/w, preferably in an amount of 0.15% w/w.

10. The liquid suspension according to any one of the preceding claims, wherein the amount of reparixin is between 2% w/w and 5.5% w/w, preferably between 3% w/w and 5.5% w/w, more preferably between 4.5% w/w and 5.5% w/w, more preferably between 4.7% w/w and 5.3% w/w, more preferably between 4.9% w/w and 5.1% w/w, even more preferably in an amount of 5% w/w.

11. The liquid suspension according to any one of claims 1 to 9, wherein the amount of reparixin is between 11.5% w/w and 12.5% w/w, preferably between 11.7% w/w and 12.3% w/w, more preferably between 11.9% w/w and 12.1% w/w, even more preferably in an amount of 12% w/w.

12. The liquid suspension according to claim 10, comprising:
- xanthan gum in an amount of 0.2% w/w,
- polyvinylpyrrolidone in an amount of 2% w/w.

13. The liquid suspension according to claim 11, comprising:
- xanthan gum in an amount of 0.2 % w/w,
- polyvinylpyrrolidone in an amount of 3% w/w.

14. The liquid suspension according to any one of the preceding claims, further comprising an aqueous solvent, preferably water, in an amount up to 100% w/w.

15. A liquid suspension according to any one of the preceding claims, for use in:
- the treatment of a disease or condition selected from psoriasis, rheumatoid arthritis, ulcerative colitis, acute respiratory distress syndrome (ARDS), idiopathic fibrosis, glomerulonephritis, COVID-19, pneumonia caused by SARS-CoV-2 or a variant thereof,
- the prevention of diabetes or in delaying the onset and the progression of diabetes, preferably Type I diabetes,
- the prevention or treatment of seizures in an individual.
